(19) **Europäisches Patentamt European Patent Office Office européen des brevets**

(11) **EP 4 614 519 A1**

(12) **EUROPEAN PATENT APPLICATION**
published in accordance with Art. 153(4) EPC

(43) Date of publication:
**10.09.2025 Bulletin 2025/37**

(51) International Patent Classification (IPC):
*G16H 50/50* (2018.01)

(21) Application number: **23885698.3**

(52) Cooperative Patent Classification (CPC):
**G16H 50/50**

(22) Date of filing: **30.10.2023**

(86) International application number:
**PCT/JP2023/039023**

(87) International publication number:
**WO 2024/095942 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.11.2022 JP 2022175512**

(71) Applicants:
• **NATIONAL UNIVERSITY CORPORATION TOKAI NATIONAL HIGHER EDUCATION AND RESEARCH SYSTEM Nagoya-shi, Aichi 464-8601 (JP)**
• **Nagoya Institute Of Technology Nagoya-shi, Aichi 466-8555 (JP)**

• **RIKEN Wako-shi, Saitama 351-0198 (JP)**

(72) Inventors:
• **TERAMACHI Ryo Nagoya-shi, Aichi 464-8601 (JP)**
• **FURUKAWA Taiki Nagoya-shi, Aichi 464-8601 (JP)**
• **KARASUYAMA Masayuki Nagoya-shi, Aichi 466-8555 (JP)**
• **YOKOTA Hideo Wako-shi, Saitama 351-0198 (JP)**

(74) Representative: **Grünecker Patent- und Rechtsanwälte PartG mbB Leopoldstraße 4 80802 München (DE)**

(54) **INFORMATION PROCESSING DEVICE, INFORMATION PROCESSING METHOD, AND COMPUTER PROGRAM**

(57) This information processing device is for predicting the prognosis of a subject patient affected by a disease, and includes a model acquisition unit, a subject patient information acquisition unit, and a prognosis prediction execution unit. The model acquisition unit acquires a prognosis prediction model, being a machine learning model that takes time-series information indicating a time-series transition of factors of the disease as an input, and outputs a prognosis of the disease. The subject patient information acquisition unit acquires time-series information about the subject patient. The prognosis prediction execution unit executes prognosis prediction of the subject patient using the time-series information about the subject patient and the prognosis prediction model, and outputs a result of the prognosis prediction.

EP 4 614 519 A1

| | PATIENT BACKGROUND | EXAMINATION FINDINGS | ENVIRONMENTAL FACTORS

NO$_2$
PM$_{2.5}$
⋮
TEMPERATURE
⋮ | TREATMENT INFORMATION | ⋯ |
|---|---|---|---|---|---|
| FIRST MONTH DATA | | | | | |
| SECOND MONTH DATA | | | | | |
| ⋮ | | | | | |
| MTH MONTH DATA | | | | | |

PROGNOSIS PREDICTION MODEL — MO

STATE IN (M+N)TH MONTH

SURVIVAL     ACUTE EXACERBATION     DEATH

CUMULATIVE PROBABILITY

DEATH
ACUTE EXACERBATION

M    M+N    TIME

FIG.1

# Description

## TECHNICAL FIELD

The technique disclosed herein relates to information processing for predicting the prognosis of a patient affected by a disease.

## BACKGROUND ART

Interstitial lung disease is a general term for chronic progressive fibrotic lung diseases. Acute exacerbation of interstitial lung disease is a pathological condition in which the disease condition rapidly worsens within one month, and the prognosis is extremely poor, with an in-hospital mortality rate of approximately 50%. If acute exacerbation of interstitial lung disease can be predicted with a high accuracy, it would become possible, for example, to suppress onset with antifibrotic drugs, or to improve the prognosis through early diagnosis and therapeutic intervention.

Conventionally, a clinical model has been proposed for predicting the risk of acute exacerbation in patients with idiopathic pulmonary fibrosis, which is a classification of interstitial lung disease (see, for example, Non-Patent Literature 1).

## CITATION LIST

### Non-Patent Literature

Non-Patent Literature 1: Qi Wu, and 5 others, "A Clinical Model for the Prediction of Acute Exacerbation Risk in Patients with Idiopathic Pulmonary Fibrosis", BioMed Research International, Hindawi, 2020, p. 1-6.

## SUMMARY OF INVENTION

### TECHNICAL PROBLEM

The progression of interstitial lung disease varies from patient to patient, and a patient's condition changes over time. In the conventional prediction model mentioned above, time-series transitions of the factors of the disease, including the patient's condition, are not taken into account, and as a result, there is a problem in that the prediction accuracy is low. Such a problem is not limited to prediction of acute exacerbation of interstitial lung disease, but is a problem common to disease prognosis prediction in general.

Herein, a technique capable of solving the problem described above will be disclosed.

### SOLUTION TO PROBLEM

The technique disclosed herein can be realized, for example, as the following aspects.
(1) An information processing device disclosed herein is a device for predicting a prognosis of a subject patient affected by a disease, and comprises a model acquisition unit, a subject patient information acquisition unit, and a prognosis prediction execution unit. The model acquisition unit acquires a prognosis prediction model, being a machine learning model that takes time-series information indicating a time-series transition of factors of the disease as an input, and outputs a prognosis of the disease. The subject patient information acquisition unit acquires time-series information about the subject patient. The prognosis prediction execution unit executes prognosis prediction of the subject patient using the time-series information about the subject patient and the prognosis prediction model, and outputs a result of the prognosis prediction.

According to this information processing device, it is possible to predict the prognosis of a disease for each individual patient based on time-series information indicating a time-series transitions of the factors of the disease, and it is possible to predict the prognosis of the disease with high accuracy.

(2) The information processing device described above may be configured such that the factors of the disease include an environmental factor. According to this configuration, by using information indicating a time-series transition of an environmental factor that could have a significant effect on the prognosis of the disease, the prognosis of the disease can be predicted with higher accuracy.

(3) The information processing device described above may be configured such that the factors of the disease include an environmental factor of a place of residence of the subject patient. According to this configuration, for example, compared to a case where an environmental factor of the location of the hospital that the subject patient visits is used, by using information indicating a time-series transition of a factor relating to the environment to which the subject patient is primarily exposed, the prognosis of the disease can be predicted with even higher accuracy.

(4) The information processing device described above may be configured such that the environmental factor of the place of residence of the subject patient is an environmental factor of a point within a straight line distance of 200 km from a current address of the subject patient. According to this configuration, by using an environmental factor relating to a relatively close point from the current address of the subject patient, it is possible to use information that more accurately indicates a time-series transition of a factor of the environment to which the subject patient is primarily exposed, and the prognosis of the disease can be predicted with extremely high accuracy.

(5) The information processing device described above may be configured such that the environmental factor includes at least one of a presence status of an environmental pollutant, and a meteorological parameter. According to this configuration, by using information indicating a time-series transition of an environmental factor that could have a significant effect on the prognosis of the disease, the prognosis of the disease can be predicted with even higher accuracy.

(6) The information processing device described above may be configured such that the time-series information includes information indicating an amount of change in the environmental factor. According to this configuration, by using information indicating an amount of change in an environmental factor that could have a significant effect on the prognosis of the disease, the prognosis of the disease can be predicted with even higher accuracy.

(7) The information processing device described above may be configured such that the time-series information is information that specifies values of the factors of the disease at a fixed time interval. According to this configuration, compared to a case where information that irregularly specifies values of the factors of the disease is used, the prognosis of the disease can be predicted with even higher accuracy.

(8) The information processing device described above may be configured such that the time-series information includes information that specifies values of the factors of the disease at least every month. According to this configuration, by using time-series information indicating monthly changes in the factors of the disease, the prognosis of the disease can be predicted with even higher accuracy.

(9) The information processing device described above may be configured such that the prognosis of the disease includes an occurrence of a plurality of events that are in a competing risk relationship, and the prognosis prediction model is a model trained using a machine learning algorithm corresponding to the plurality of events that are in a competing risk relationship. According to this configuration, the occurrence of a plurality of events that are in a competing risk relationship can be predicted with high accuracy.

(10) The information processing device described above may be configured such that the prognosis prediction model outputs, as the prognosis of the disease, an index value representing a possibility of an event occurring among the plurality of events that are in a competing risk relationship. According to this configuration, the occurrence of a plurality of events that are in a competing risk relationship can be predicted with high accuracy.

(11) The information processing device described above may be configured such that the plurality of events that are in a competing risk relationship include acute exacerbation and death. According to this configuration, the occurrence of each of acute exacerbation and death, which are in a competing risk relationship, can be predicted with high accuracy.

(12) The information processing device described above may be configured such that the disease is a disease of a respiratory system or a circulatory system. According to this configuration, by using time-series information indicating a time-series transition of the factors of a disease of the respiratory system or the circulatory system, it is possible to predict the prognosis of a disease of the respiratory system or the circulatory system with high accuracy.

(13) The information processing device described above may be configured such that the prognosis prediction execution unit executes a hypothetical prognosis prediction of the subject patient using hypothetical information, in which a portion of the time-series information about the subject patient has been changed, and the prognosis prediction model, and predicts an effect of an intervention corresponding to the change based on an actual prognosis prediction result and a hypothetical prognosis prediction result, and outputs a prediction result of the effect of the intervention. According to this configuration, it is possible to determine whether or not to actually perform an intervention based on an output prediction result of the effect of the intervention.

The technique disclosed herein can be realized in various forms, such as an information processing device, an information processing method, a computer program that implements such a method, and a non-transitory recording medium that records such a computer program.

BRIEF DESCRIPTION OF DRAWINGS

FIG. 1 is an explanatory diagram schematically showing a prognosis prediction model MO according to the present embodiment.
FIG. 2 is an explanatory diagram showing a prediction method of the effect of an intervention based on a prognosis prediction result.
FIG. 3 is an explanatory diagram showing a schematic configuration of an information processing device 100.
FIG. 4 is a flowchart showing prognosis prediction model acquisition processing in the present embodiment.
FIG. 5 is an explanatory diagram showing a specific example of factors (feature amounts) of interstitial lung disease.

FIG. 6 is an explanatory diagram schematically showing estimation processing performed with respect to raw information Io.

FIG. 7 is an explanatory diagram showing an example of learning data LD obtained via preprocessing.

FIG. 8 is an explanatory diagram schematically showing a model using LSTM.

FIG. 9 is a flowchart showing prognosis prediction processing in the present embodiment.

FIG. 10 is an explanatory diagram showing the prediction accuracy of the prognosis prediction model MO according to an example.

FIG. 11 is another explanatory diagram showing the prediction accuracy of the prognosis prediction model MO according to an example.

FIG. 12 is another explanatory diagram showing the prediction accuracy of the prognosis prediction model MO according to an example.

FIG. 13 is an explanatory diagram showing the prediction accuracy of the prognosis prediction model MO according to an example.

FIG. 14 is an explanatory diagram showing an example of a prognosis prediction result by the prognosis prediction model MO according to an example.

FIG. 15 is an explanatory diagram showing the prediction accuracy of the prognosis prediction model MO according to another example.

FIG. 16 is an explanatory diagram showing an example of the importance of each factor of a disease according to an example.

FIG. 17 is an explanatory diagram showing an example of the importance of each factor of a disease according to another example.

FIG. 18 is an explanatory diagram showing the relationship between the upper limit value of a straight line distance from the current address of a patient to a measurement station and the prediction accuracy.

FIG. 19 is an explanatory diagram showing the relationship between the upper limit value of a straight line distance from the current address of a patient to a measurement station and the prediction accuracy.

EMBODIMENTS OF THE INVENTION

A. Embodiment:

A-1: Overview of Prognosis Prediction Model MO:

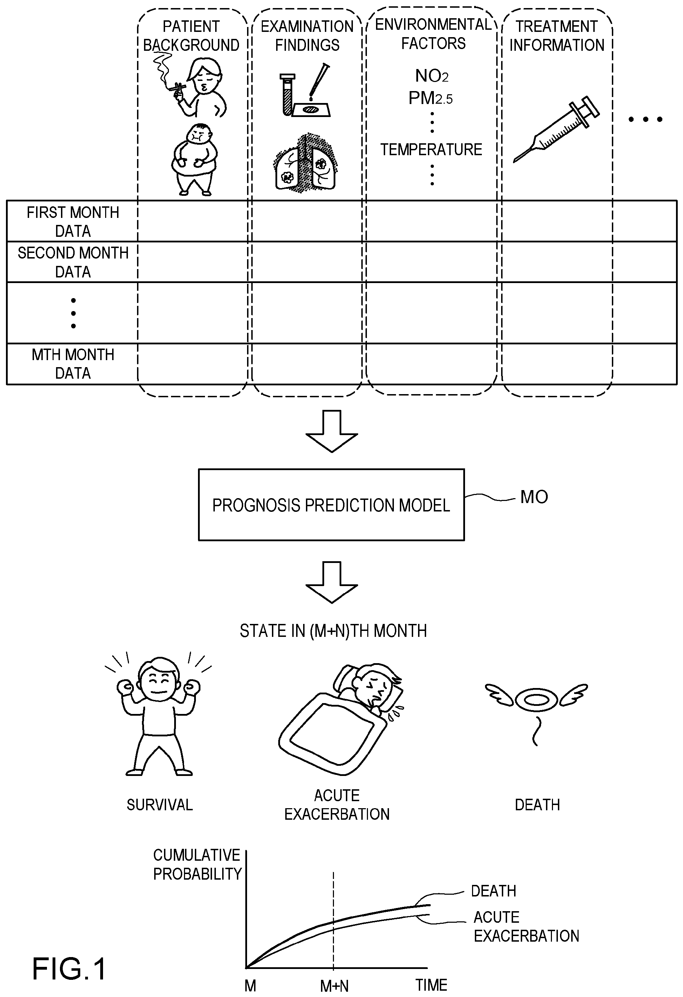

First, an overview of a prognosis prediction model MO according to the present embodiment will be described. FIG. 1 is an explanatory diagram schematically showing a prognosis prediction model MO according to the present embodiment. The prognosis prediction model MO is a model for predicting the prognosis of a patient affected by a disease. The prognosis prediction model MO is a machine learning model that takes time-series information indicating a time-series transition of factors (feature amounts) of the disease as an input, and outputs a prognosis of the disease. Herein, machine learning refers to a general term for techniques and methods that derive rules and patterns by using a computer to learn based on large amounts of data (that is, data-driven learning), and includes deep learning.

In the present embodiment, interstitial lung disease will be used as a specific example of a disease. Examples of events of interstitial lung disease include acute exacerbation and death. Because an acute exacerbation event does not occur after a death event, the two events can be said to be in a competing risk relationship.

Examples of factors (feature amounts) of interstitial lung disease include patient background (smoker or non-smoker, BMI, and the like), examination findings (blood examinations, and chest CT images, and the like), environmental factors (environmental contaminants such as $NO_2$ and $PM_{2.5}$, meteorological parameters such as temperature, and the like), and treatment information (administration of antifibrotic agents and the like). The time-series information indicating a time-series transition of such factors is information that specifies, for example, over a certain time period (for example, a time period up to the Mth month from an initial diagnosis), the values or amounts of change in the factors at fixed time intervals (for example, monthly).

As the prognosis prediction performed by the prognosis prediction model MO, for example, an index value that indicates the possibility of each event occurring is calculated. In the example shown in FIG. 1, as the prognosis prediction, the probability of acute exacerbation and death occurring at a certain timing (for example, in the (M+N)th month) is calculated. However, the prognosis prediction may be executed in another form. For example, as the prognosis prediction, a classification of the predicted state (survival, acute exacerbation, or death) of the patient may be performed based on the probability of acute exacerbation and death occurring at a certain timing.

As a result of using the prognosis prediction model MO of the present embodiment, it is possible to accurately predict, for individual patients, the prognosis of the patient based on time-series information indicating time-series transitions of the factors of the disease. The prognosis prediction result can be used in various applications. For example, for a patient in which it has been predicted that the probability of developing acute exacerbation is high, it is possible to administer antifibrotic drugs to suppress the onset of the disease, or to improve the prognosis by carrying out early diagnosis and therapeutic intervention.

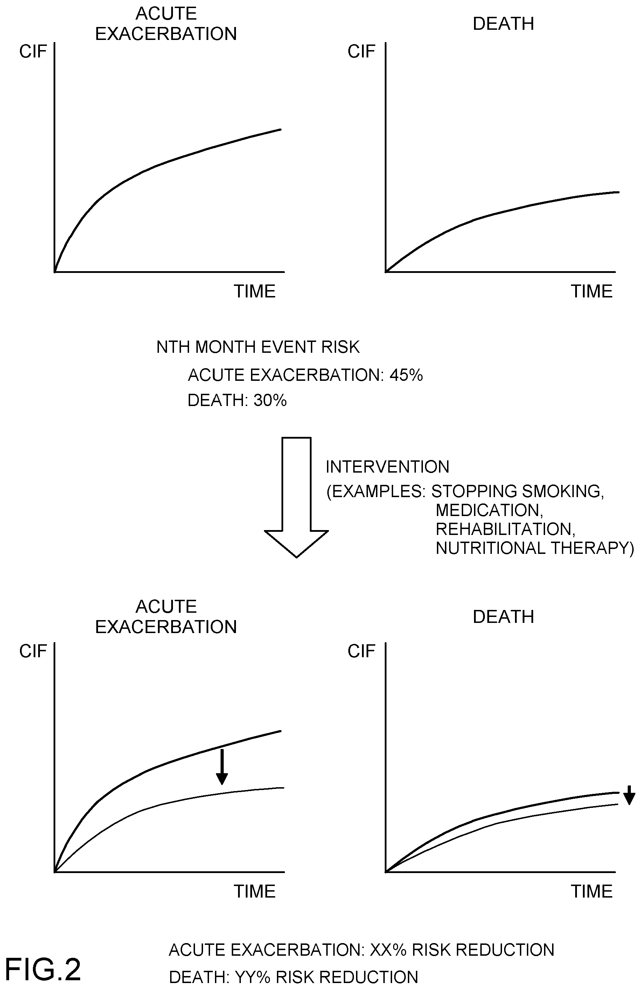

As shown in FIG. 2, it is possible to predict the effect of an intervention based on the prognosis prediction result. The upper part of FIG. 2 shows an example of prediction results of the probability of acute exacerbation and death occurring based on actual time-series information. The lower part of FIG. 2 shows an example of a hypothetical prediction result of the probability of acute exacerbation and death occurring based on hypothetical information in which a portion of the time-series information has been changed so as to correspond to an expected intervention (such as stopping smoking, medication, rehabilitation, or nutritional therapy). Based on the prognosis prediction results according to the actual time-series information and the prognosis prediction results according to the hypothetical information, the effect (such as an XX% reduction in the risk of acute exacerbation, and a YY% reduction in the risk of death) of the intervention corresponding to the change described above can be predicted. It is possible to determine whether or not to actually perform the intervention based on the output prediction result of the effect of the intervention.

A-2. Configuration of Information Processing Device 100:

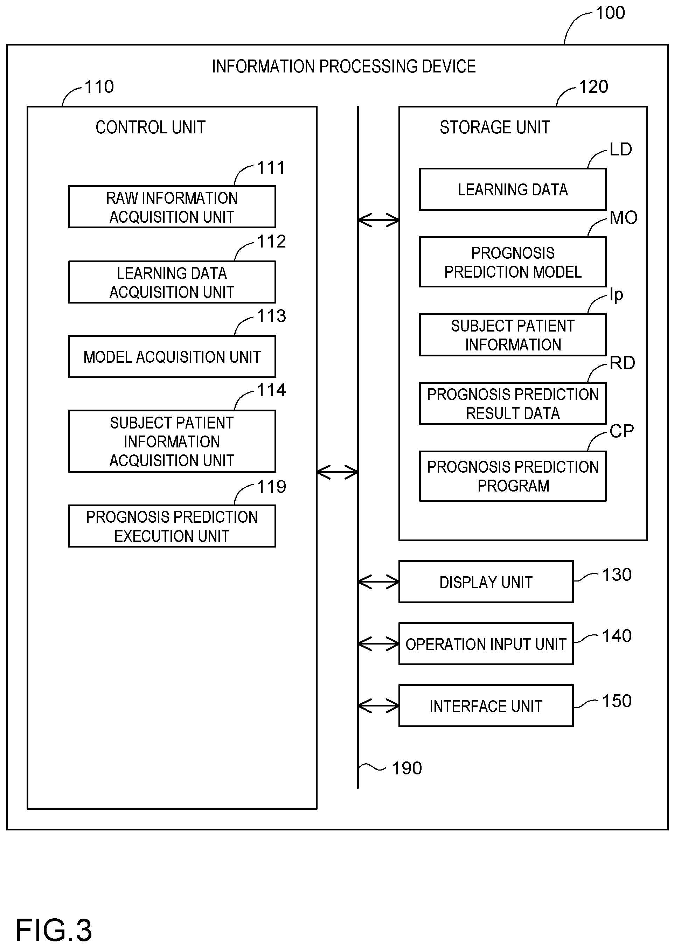

Next, the configuration of an information processing device 100 for creating a prognosis prediction model MO and executing a prognosis prediction using the prognosis prediction model MO will be described. FIG. 3 is an explanatory diagram showing a schematic configuration of the information processing device 100. The information processing device 100 is configured by a computer (a PC, a server, or the like).

The information processing device 100 includes a control unit 110, a storage unit 120, a display unit 130, an operation input unit 140, and an interface unit 150. Each of these units are connected so as to be capable of communicating with each other via a bus 190. The information processing device 100 may include a speaker serving as an output means.

The display unit 130 of the information processing device 100, for example, is configured by a liquid crystal display, and displays various images and information. The operation input unit 140, for example, is configured by a keyboard, a mouse, a button, a microphone, or a trackpad, and receives operations and instructions from an administrator. The display unit 130 may also be provided with a touch panel, and function as the operation input unit 140. The interface unit 150, for example, is configured by a LAN interface or a USB interface, and communicates with other devices in a wired or wireless fashion.

The storage unit 120 of the information processing device 100 is configured by, for example, a ROM, a RAM, a hard disk drive (HDD), or the like, stores various programs and data, and is used as a work area when the various programs are executed, and as a temporary storage area for data. For example, the storage unit 120 stores a prognosis prediction program CP, which is a computer program for executing the prognosis prediction model acquisition processing and prognosis prediction processing described below. The prognosis prediction program CP is provided, for example, in a state where the program is stored in a computer-readable recording medium such as a CD-ROM, DVD-ROM, or USB memory (not shown), or is provided in a state where the program can be acquired from an external device (a server or other terminal device on a network) via the interface unit 150, and is stored in the storage unit 120 in a state where the program can operate on the information processing device 100.

The storage unit 120 of the information processing device 100 stores, in advance, or during execution of the prognosis prediction model acquisition processing and the prognosis prediction processing described below, learning data LD, the prognosis prediction model MO, subject patient information Ip, and prognosis prediction result data RD. The content of such information and data will be described together with the description of the prognosis prediction model acquisition processing and the prognosis prediction processing described below.

The control unit 110 of the information processing device 100 is configured, for example, by a CPU or the like, and controls the operation of the information processing device 100 by executing the computer program that has been read from the storage unit 120. For example, as a result of reading and executing the prognosis prediction program CP from the storage unit 120, the control unit 110 functions as a raw information acquisition unit 111, a learning data acquisition unit 112, a model acquisition unit 113, a subject patient information acquisition unit 114, and a prognosis prediction execution unit 119 for executing the prognosis prediction model acquisition processing and the prognosis prediction processing described below. The function of each of these units will be described together with the description of the prognosis prediction model acquisition processing and the prognosis prediction processing described below.

A-3. Prognosis Prediction Model Acquisition Processing:

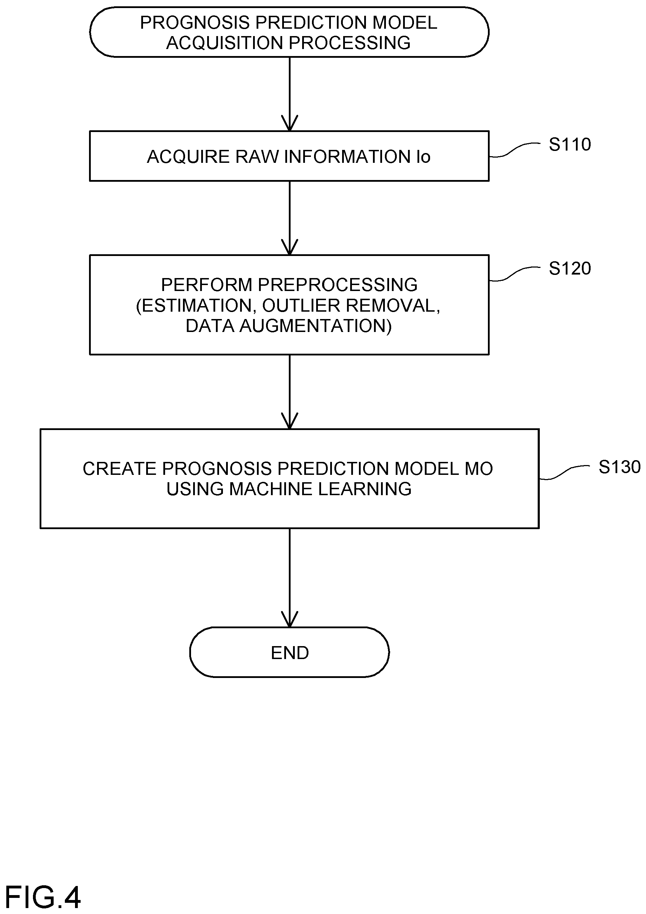

Next, the prognosis prediction model acquisition processing that is executed by the information processing device 100 according to the present embodiment will be described. FIG. 4 is a flowchart showing the prognosis prediction model acquisition processing in the present embodiment. The prognosis prediction model acquisition processing is processing that acquires the prognosis prediction model MO, which is a machine learning model for predicting the prognosis of a patient affected by a disease (interstitial lung disease). In the present embodiment, the information processing device 100 creates the prognosis prediction model MO itself by performing a predetermined machine learning to acquire the prognosis prediction model MO. The prognosis prediction model acquisition processing is started in response to a user inputting a start instruction by operating the operation input unit 140 of the information processing device 100.

First, the raw information acquisition unit 111 of the information processing device 100 (FIG. 3) acquires information (hereinafter, referred to as "raw information Io") used to create the prognosis prediction model MO (S110). The raw information Io is information that serves as the basis of the learning data LD used to train, validate, and test the prognosis prediction model MO. Specifically, the raw information Io is information in which time-series information indicating time-series transitions of the factors (feature amounts) of interstitial lung disease and information indicating the prognosis are associated with each other for a plurality of patients affected by interstitial lung disease. The raw information Io is acquired via the interface unit 150, or via the operation input unit 140.

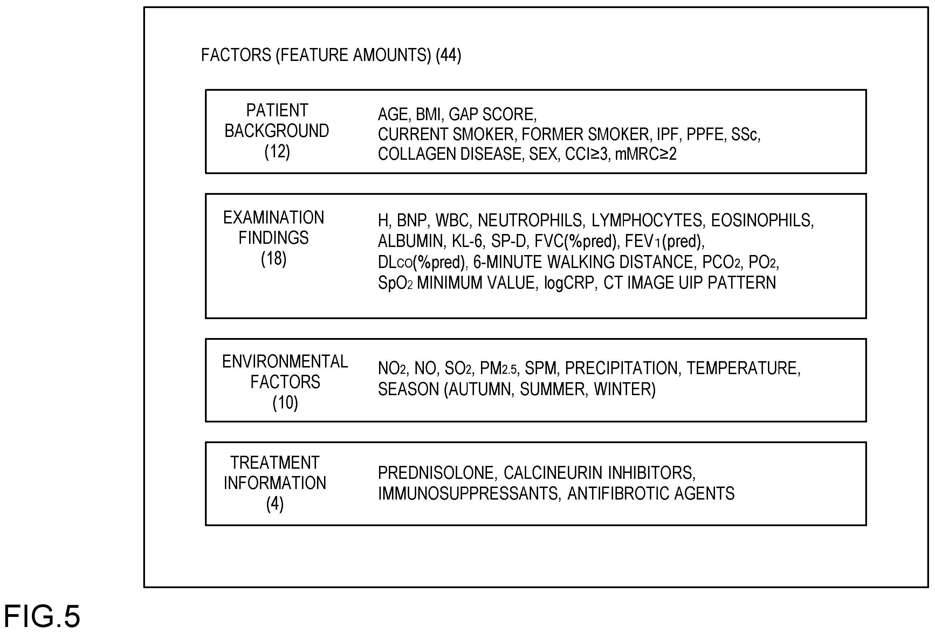

FIG. 5 is an explanatory diagram showing a specific example of factors (feature amounts) of interstitial lung disease. In the present embodiment, 44 factors are used as factors of interstitial lung disease, which are classified into four types, namely patient background, examination findings, environmental factors, and treatment information.

The patient background, for example, includes the following 12 factors. The information about the patient background is obtained, for example, through medical questionnaires and examinations.

- Age, BMI, GAP score, current smoker, former smoker, IPF, PPFE, SSc, collagen disease, sex, $CCI \geq 3$, $mMRC \geq 2$

The examination findings include, for example, the following 18 factors. The information about the examination findings is obtained, for example, from blood examinations and a chest CT images.

- LDH, BNP, WBC, neutrophils, lymphocytes, eosinophils, albumin, KL-6, SP-D, FVC (%pred), $FEV_1$ (%pred), DLco (%pred), 6-minute walking distance, $PCO_2$, $PO_2$, $SpO_2$ minimum value, log CRP, CT image UIP pattern

The environmental factors are factors relating to the environment of the place of residence of the patient, and for example, include the presence status (such as the concentration) of environmental pollutants, and meteorological parameters. More specifically, the environmental factors include, for example, the following 10 factors.

- $NO_2$, NO, $SO_2$, $PM_{2.5}$, SPM, precipitation, temperature, season (autumn, summer, winter)

As the environmental factors, it is possible to use a representative value over a fixed period of time (such as a monthly average value or a daily average value), the amount of change over a fixed period of time (such as an amount of change in a monthly average value, an amount of change in a daily average value), and/or the number of times that a value exceeded the reference value over a fixed period of time (such as the number of days that an environmental value exceeded the reference value per month, or the number of hours that an environmental value exceeded the reference value per day).

The place of residence of the patient may be an area to which the current address of the patient belongs. In this case, the information among the environmental factors relating to environmental pollutants is obtained, for example, by referring to measurement data from a measurement station located in the area. Specifically, the measurement data from the measurement station whose straight line distance is the closest to the current address of the patient is referenced. In a case where a measurement station is not present in the range of a predetermined upper limit distance (such as 100 km) from the current address of the patient, there is a data absence in the environmental factors obtained by referencing the measurement data of a measurement station. The measurement station data can be acquired, for example, from the National Institute for Environmental Studies website. Among the environmental factors, information relating to meteorological parameters (precipitation, temperature, and season) can be obtained, for example, from the Japan Meteorological Agency website.

The place of residence of the patient may also be the room in which the patient lives (such as inside a clean room). In this case, at least a portion of the information relating to the environmental factors is obtained, for example, by referring to measurement data obtained by a sensor installed in the room, or a sensor attached to the patient.

The information relating to the environmental factors may be acquired by referring to measurement values from man-made satellites or predicted values from weather simulations.

The treatment information includes, for example, the following 4 factors. The treatment information is obtained, for example, from records of treatment results.

- Prednisolone, calcineurin inhibitors, immunosuppressants, antifibrotic agents

Next, as a result of the learning data acquisition unit 112 of the information processing device 100 (FIG. 3) performing preprocessing of the raw information Io, the learning data LD is acquired (S120 of FIG. 4). As the preprocessing, for example, estimation, outlier removal, and data augmentation, are executed.

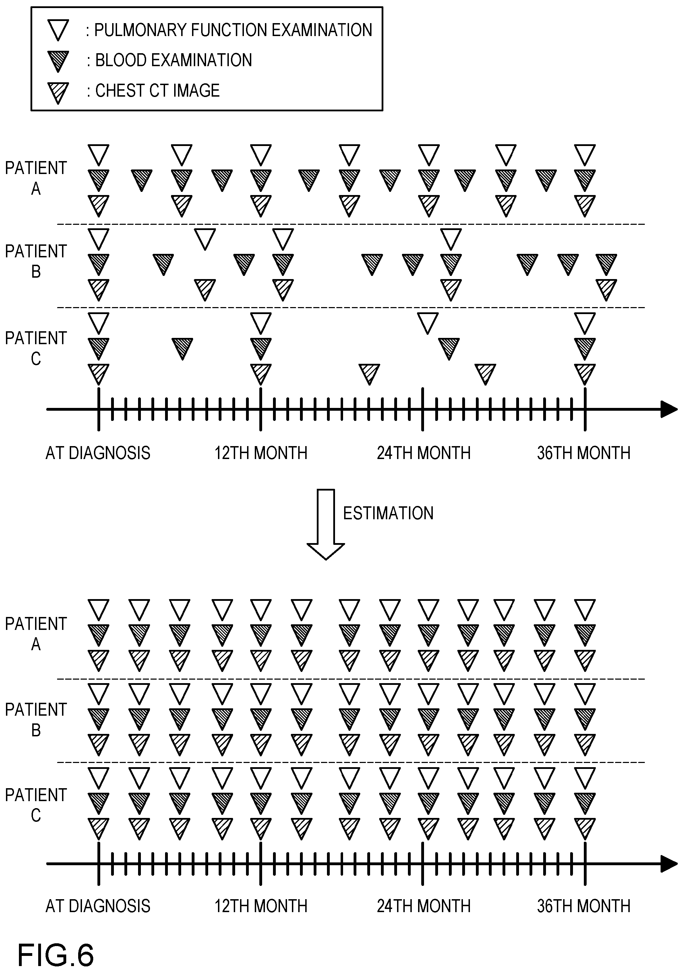

FIG. 6 is an explanatory diagram schematically showing estimation processing performed with respect to raw information Io. As shown in the upper part of FIG. 6, before estimation processing, the timing of the data relating to the factors obtained from each examination (such as pulmonary function examinations, blood examinations, and chest CT images) varies due to the fact that the examinations for each patient are performed at different times. In the present embodiment, estimation processing is performed so that the data at fixed time intervals is obtained for all of the factors. The same applies to factors other than those obtained by examinations.

When performing the estimation processing, it is preferable to select and execute estimation processing that matches the characteristics of each factor. For example, for factors that are obtained from examination items that are similar to each other, the estimation is performed using multiple regression analysis based on a group created from the similar examination items. For factors that can be estimated with a high probability from the preceding or subsequent values, the estimation is performed using linear estimation (interpolation) or nearest neighbor estimation (extrapolation). For factors that exhibit rapid fluctuations in a short period of time (such as CRP), the estimation is performed using nearest neighbor estimation (interpolation or extrapolation). For categorical variables, the estimation is performed using nearest neighbor estimation (interpolation or extrapolation). For factors that have a low correlation between time and the data (such as environmental pollutants), a data absence can be set without performing estimation. As a result of performing such estimation processing, data having a fixed time interval can be obtained for each factor without losing the characteristics of each factor.

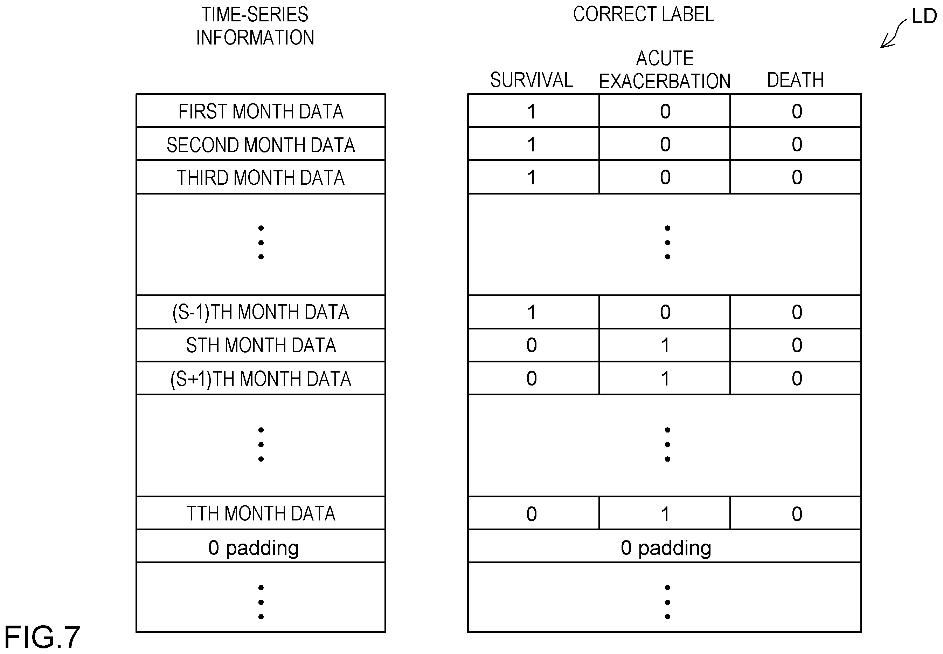

FIG. 7 is an explanatory diagram showing an example of learning data LD obtained via preprocessing. The learning data LD is data in which time-series information (monthly data in the example of FIG. 7) indicating time-series transitions of each factor (feature amount) and information (correct label) indicating the prognosis at each timing are associated with each other. FIG. 7 shows data for a patient that developed acute exacerbation in the tth month after initial diagnosis. In a case where development of acute exacerbation is predicted before the (t-s)th month, in the data for the patient, the "survival" label is "1" from the first month until the (s-1)th month, and the remaining labels are "0", and after the sth month, the "acute exacerbation" label is "1", and the remaining labels are "0". After the tth month, the data is zero-padded.

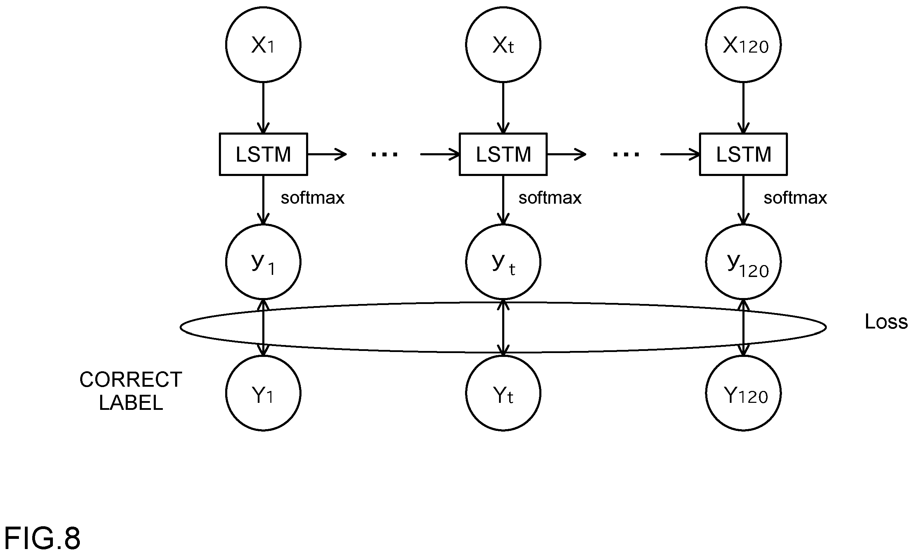

Then, the model acquisition unit 113 of the information processing device 100 (FIG. 3) creates the prognosis prediction model MO by machine learning using the learning data LD (S130 of FIG. 4). It is possible to use various known machine learning algorithms as the machine learning for creating the prognosis prediction model MO. For example, LSTM (long short term memory) may be used to create the prognosis prediction model MO. FIG. 8 is an explanatory diagram schematically showing a model using LSTM. LSTM is an improvement of a recurrent neural network (RNN), which is capable of handling time-series information, in order to solve the vanishing gradient problem. As shown in FIG. 8, in creating the prognosis prediction model MO using LSTM, the model is updated so as to reduce the loss calculated from the output value $y_t$ and the correct label $Y_t$ when the feature amount $X_t$ of the tth month is input.

Alternatively, the Dynamic-DeepHit model may be used to create the prognosis prediction model MO. The Dynamic-DeepHit model is a known machine learning algorithm that supports a plurality of events that are in a competing risk relationship. As mentioned above, because acute exacerbation and death, which are events of interstitial lung disease, are in a competing risk relationship, if a machine learning algorithm that corresponds to a plurality of events in a competing risk relationship is used, it is possible to create a prognosis prediction model MO with high prediction accuracy. Details of the Dynamic-DeepHit model are described in, for example, the following document.

- Lee Changhee, and 3 others, "DeepHit: A Deep Learning Approach to Survival Analysis with Competing Risks", Proceedings of the 31st AAAI Conference on Artificial Intelligence, Association for the Advancement of Artificial Intelligence, 2018, p. 2314-2321.

The prognosis prediction model MO created by machine learning is stored in the storage unit 120 of the information processing device 100. This completes the acquisition processing of the prognosis prediction model MO (FIG. 4). When creating the prognosis prediction model MO, for example, a portion of the learning data LD is used as training data for updating the parameters (weights and the like) of the model, another portion of the learning data LD is used as validation data for setting a hyperparameter, and another portion of the learning data LD is used as test data to confirm the generalization performance of the model.

A-4. Prognosis Prediction Processing:

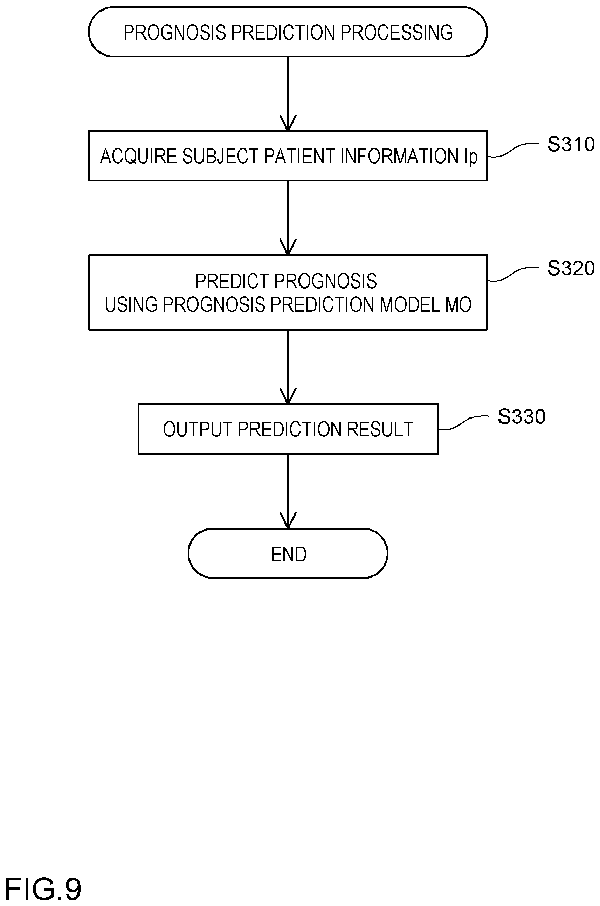

Next, the prognosis prediction processing that is executed by the information processing device 100 according to the present embodiment will be described. FIG. 9 is a flowchart showing the prognosis prediction processing in the present embodiment. The prognosis prediction processing is processing in which the prognosis prediction model MO is used to perform prognosis prediction (predict the risk of acute exacerbation or death) of a patient affected by interstitial lung disease. The prognosis prediction processing is started in response to a user inputting a start instruction by operating the operation input unit 140 of the information processing device 100.

First, the subject patient information acquisition unit 114 of the information processing device 100 (FIG. 3) acquires the subject patient information Ip (S310). The subject patient information Ip is the time-series information described above for the patient subjected to prognosis prediction processing. The subject patient information Ip is acquired via the interface unit 150, or via the operation input unit 140, and is stored in the storage unit 120.

Then, the prognosis prediction execution unit 119 of the information processing device 100 (FIG. 3) uses the subject patient information Ip and the prognosis prediction model MO, and executes prognosis prediction of the subject patient (S320). That is, the prognosis prediction execution unit 119 acquires the prognosis prediction result that is output from the prognosis prediction model MO by inputting the subject patient information Ip to the prognosis prediction model MO. The prognosis prediction execution unit 119 generates the prognosis prediction result data RD, which is information indicating the prognosis prediction result, and stores the data in the storage unit 120 of the information processing device 100.

Then, the prognosis prediction execution unit 119 outputs a prognosis prediction result based on the prognosis prediction result data RD (S330). For example, the prognosis prediction execution unit 119 causes the display unit 130 to display the prognosis prediction result. This completes the prognosis prediction processing.

For example, a physician or the like can refer to the displayed prognosis prediction result, and for a patient in which it has been predicted that the probability of developing acute exacerbation is high, administer antifibrotic drugs to suppress the onset of the disease, or improve the prognosis by carrying out early diagnosis and therapeutic intervention.

As shown in FIG. 2, the prognosis prediction execution unit 119 executes a hypothetical prognosis prediction of the subject patient using hypothetical information, in which a portion of the time-series information for the subject patient has been changed, and the prognosis prediction model MO, and predicts the effect of an intervention corresponding to the change based on an actual prognosis prediction result and a hypothetical prognosis prediction result, and outputs a prediction result of the effect of the intervention. In this way, it is possible to determine whether or not to actually perform an intervention based on an output prediction result of the effect of the intervention.

A-5. Example:

An example of the prognosis prediction model MO mentioned above will be described below. The prognosis prediction model MO of the present example was created through a multicenter, retrospective study of patients with newly diagnosed interstitial lung disease at two hospitals (Tosei Public Hospital and Hamamatsu University School of Medicine) from 2008 to 2015. Of the 839 cases from the Tosei Public Hospital, 80% were used as training data for model construction, and the remaining 20% were used as validation data for internal validity verification. The 336 cases from Hamamatsu University School of Medicine were used as test data for external validity (generalization performance) verification.

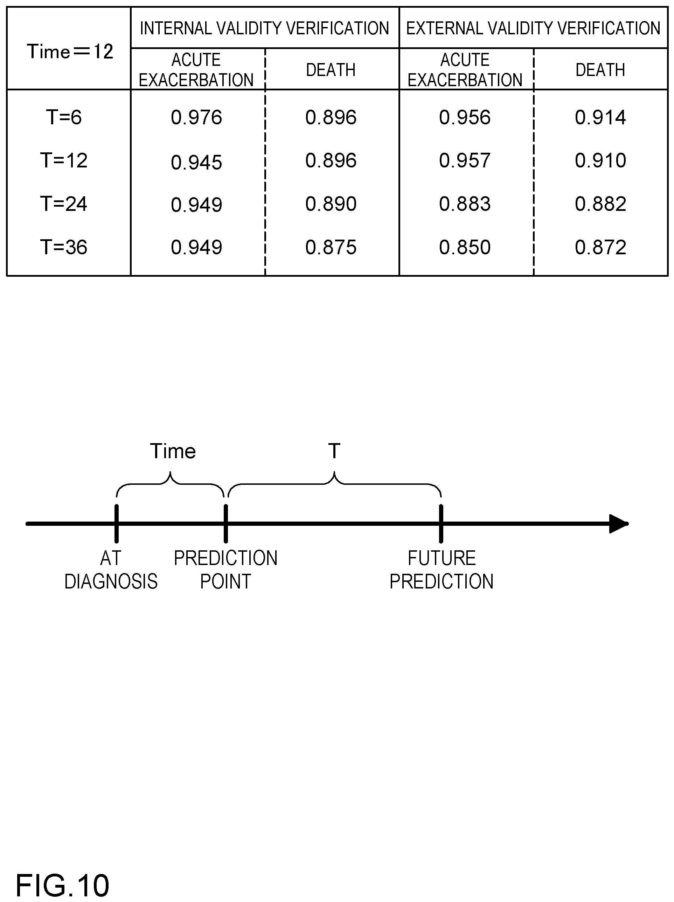

FIG. 10 is an explanatory diagram showing the prediction accuracy of the prognosis prediction model MO of according to an example. FIG. 10 shows the results of internal validity verification using validation data (C-index values) and the results of external validity verification using test data (also C-index values) for the prognosis prediction model MO created using the Dynamic-DeepHit model described above. In the example of FIG. 10, the accuracy of the prognosis prediction result T months later (T = 6, 12, 24, 36) is shown when the prediction time is set to 12 months after the initial diagnosis. As shown in FIG. 10, a high C-index value of 0.85 or more was obtained for both acute exacerbation and death, and it can be said that the prognosis prediction model MO of the embodiment generally achieves high prediction accuracy. The C-index is an index that indicates prediction accuracy, where a larger value (maximum value: 1) indicates better model performance.

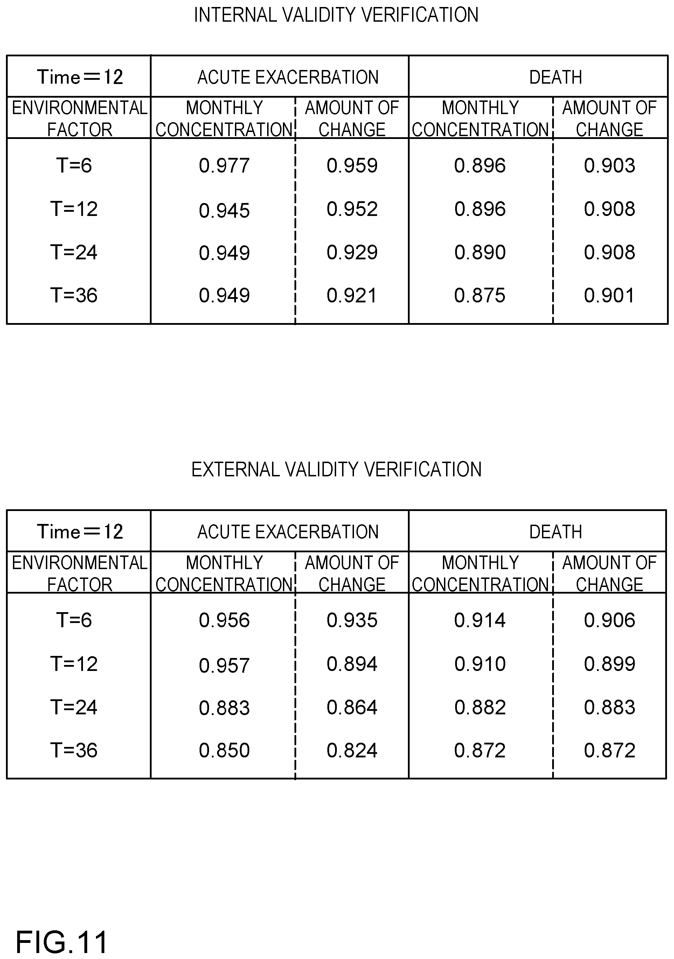

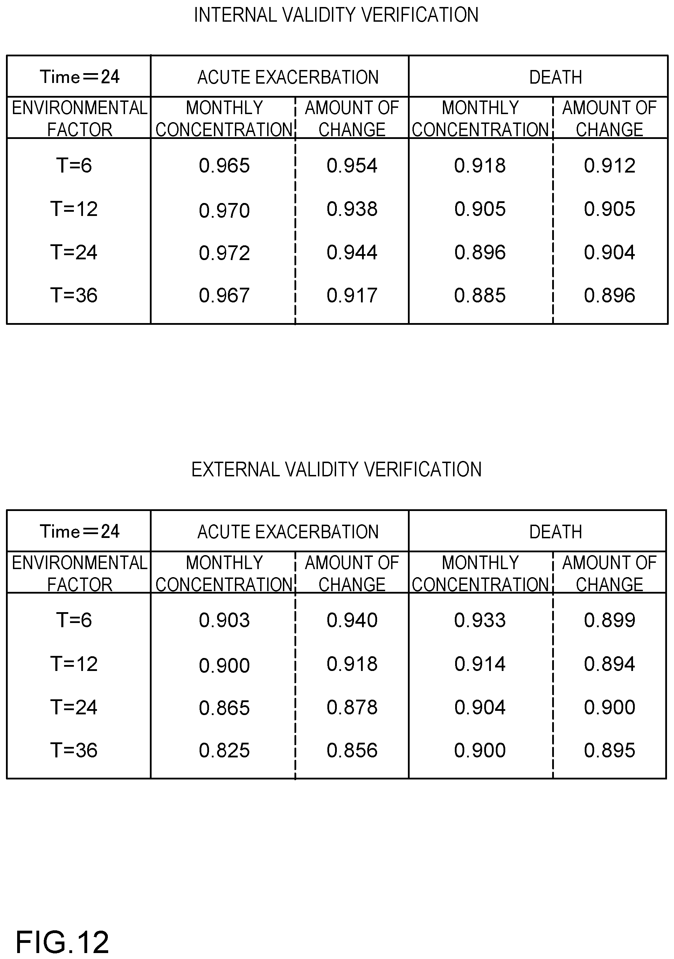

FIGS. 11 and 12 are other explanatory diagrams showing the prediction accuracy of the prognosis prediction model MO of according to an example. FIG. 11 and 12 show the results of internal validity verification (C-index values) and external validity verification (also C-index values) when a monthly concentration or an amount of change is used as the environmental factor. FIG. 11 is an example using data from the initial diagnosis up to 12 months later, and FIG. 12 is an example using data from the initial diagnosis up to 24 months later. As shown in FIGS. 11 and 12, in the example where the amount of change is used as the environmental factor, a prediction accuracy at least as high as that of the example where the monthly concentration is used as the environmental factor can be achieved.

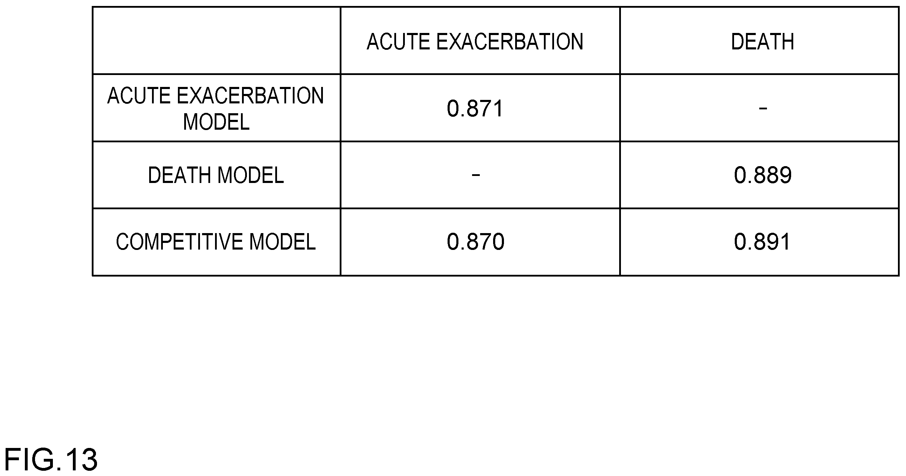

FIG. 13 is an explanatory diagram showing the prediction accuracy of the prognosis prediction model MO of according to an example. FIG. 13 shows the result of external validity verification (a C-index value) of acute exacerbation prediction by a model that predicts only acute exacerbation (an acute exacerbation model), the result of external validity verification (also a C-index value) of death prediction by a model that predicts only death (a death model), and results of external validity verification (also C-index values) of acute exacerbation and death prediction by a model that predicts both acute exacerbation and death, which are in a competing risk relationship (a competitive model), which were created using a Dynamic-DeepHit model. As shown in FIG. 13, the prediction accuracy by the model taking competing risks into account was as high as the prediction accuracy by the model taking only acute exacerbation or only death into account. Therefore, it can be said that high prediction accuracy can be achieved even with a model that takes competing risks into account.

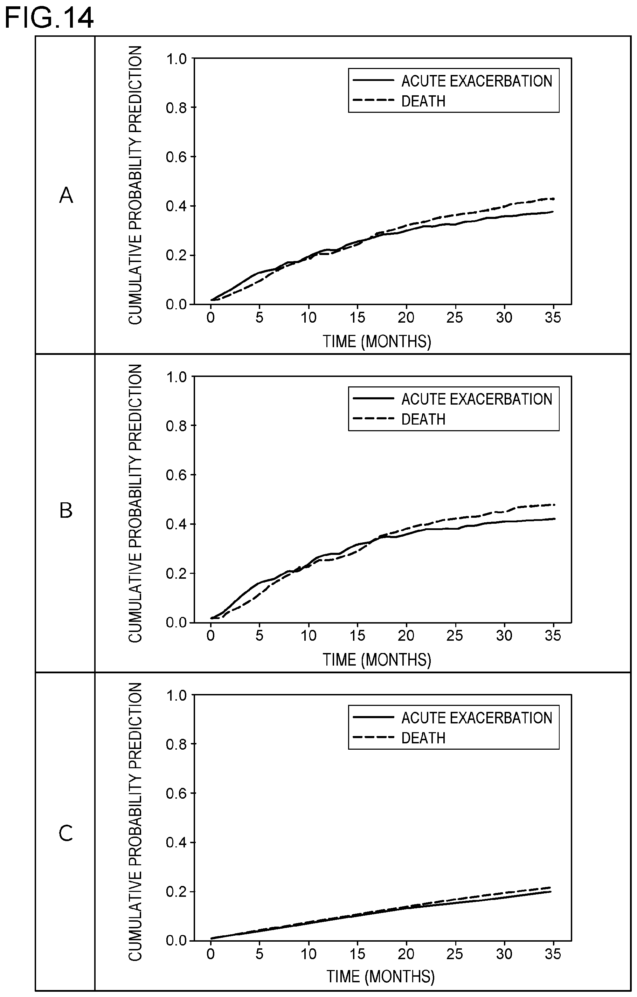

FIG. 14 is an explanatory diagram showing an example of a prognosis prediction result by the prognosis prediction model MO according to an example. FIG. 14 shows the results of predicting the cumulative occurrence probability of each event using the test data from the initial diagnosis up to 24 months, using the prognosis prediction model MO created using the Dynamic-DeepHit model. Panel A of FIG. 14 shows an example using test data in which acute exacerbation developed in the 38th month after initial diagnosis, panel B of FIG. 14 shows an example using test data in which a patient died in the 54th month after initial diagnosis, and panel C of FIG. 14 shows an example using test data in which survival was ultimately reported in the 81st month after initial diagnosis. The prediction results shown in panel B of FIG. 14 show a consistently higher probability of death compared to the example shown in panel A of FIG. 14. The prediction results shown in panel C of FIG. 14 show a consistently lower probability of both acute exacerbation and death compared to the example shown in panels A and B of FIG. 14. In this way, it can be said that the prognosis prediction model MO of the example generally achieves high prediction accuracy.

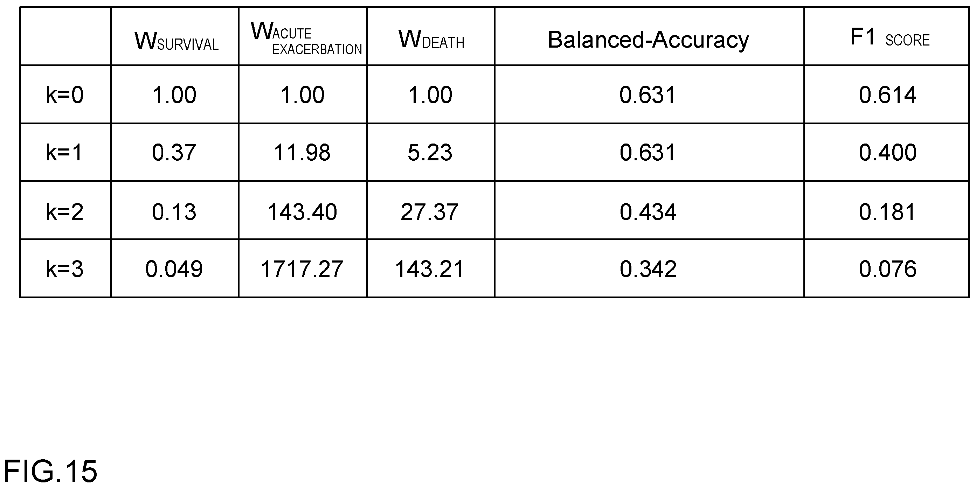

FIG. 15 is an explanatory diagram showing the prediction accuracy of the prognosis prediction model MO of according to another example. FIG. 15 shows the results of internal validation (Balanced-Accuracy and F1 score values) using validation data for the prognosis prediction model MO created using LSTM as shown in FIG. 8. In an example where the cross-entropy weighting described below is not adjusted (the example of "k = 0" in FIG. 15), the Balanced-Accuracy and F1 score values are both approximately 0.6, and a reasonable level of prediction accuracy is achieved.

Here, as shown in FIG. 7, the proportion of data in the learning data LD used to create the prognosis prediction model MO in which the correct label is "survival" is very high. That is, the learning data LD is imbalanced data. In order to correct the influence of such a data imbalance, various changes were made to the cross-entropy weights $W_j$ according to the following formula (1) to confirm the prediction accuracy of the model.

$$W_j = (N/(M \times N_j))^k \ldots (1)$$

Where,

· $W_j$: Weight of label = j
· N: Total number of labels
· M: Number of classes
· $N_j$: Count for label = j
· k: Hyperparameter

However, as shown in FIG. 15, even when the value of the hyperparameter k was adjusted to change the weight $W_j$ of each label, the prediction accuracy did not improve. Possible causes for this include, in addition to the data imbalance problem mentioned above, the problem of defining a "predicted event", such as in cases where the predicted probability of "acute exacerbation" are different from each other but are classified as the same "acute exacerbation", and the problem of the similarity between events, such as acute exacerbation, which is a fatal condition. From the above, it can be said that it is preferable to use a machine learning algorithm (such as Dynamic-DeepHit) that corresponds to a plurality of events in a competing risk relationship as described above when creating the prognosis prediction model MO for predicting acute exacerbation of interstitial lung disease and/or death.

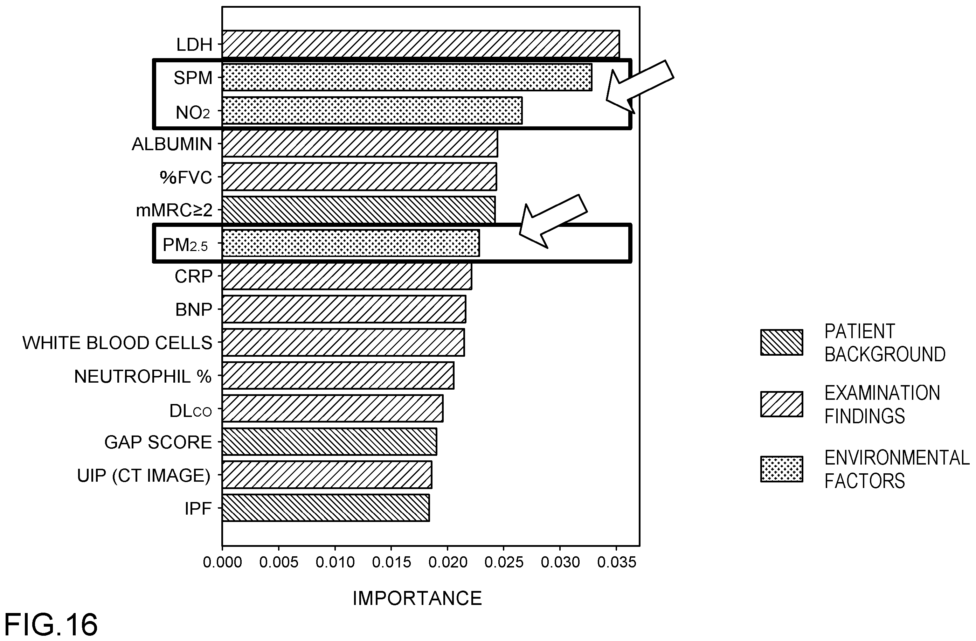

FIG. 16 is an explanatory diagram showing an example of the importance of each factor of a disease according to an example. FIG. 16 shows the importance of each factor (top 20 factors), which were obtained by examining the change in risk of acute exacerbation when the values that can be taken by each factor (feature amount) were changed, and then determining that factors having a larger change in risk have a higher importance (contribution). As shown in FIG. 16, some environmental factors (SPM, $NO_2$, and $PM_{2.5}$) were more important than factors related to lung function (FVC, DLco) and factors related to severity (GAP score). Therefore, it is preferable to use environmental factors to predict the prognosis of interstitial lung disease.

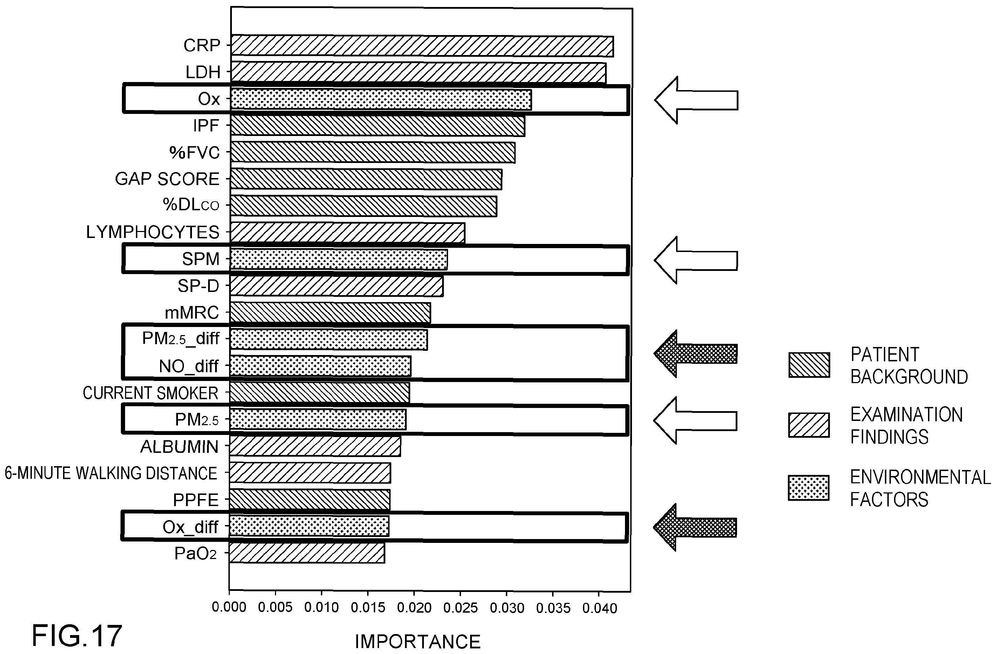

FIG. 17 is an explanatory diagram showing an example of the importance of each factor of a disease according to another example. FIG. 17 shows, in a similar fashion to FIG. 16, the importance of each factor, but in the example of FIG. 17, in addition to the monthly average value, the amount of change from the previous month (the name of the environmental factor in FIG. 17 with "diff" appended to the name) is used as the environmental factor. As shown in FIG. 17, for some environmental factors ($PM_{2.5}$, NO, and Ox), the importance of the change from the previous month is high. Therefore, it is preferable to use the amount of change for each fixed period of time as the environmental factor used to predict the prognosis of interstitial lung disease, in addition to, or instead of, the representative value for each fixed period of time. From the results shown in FIG. 17, it can be said that there is a difference between environmental factors in terms of whether the representative value for each certain period is important, or the amount of change is important. For example, for SPM, the representative value for each fixed period has a high importance, for NO, the amount of change has a high importance, and for both $PM_{2.5}$ and Ox, the importance of both values is high.

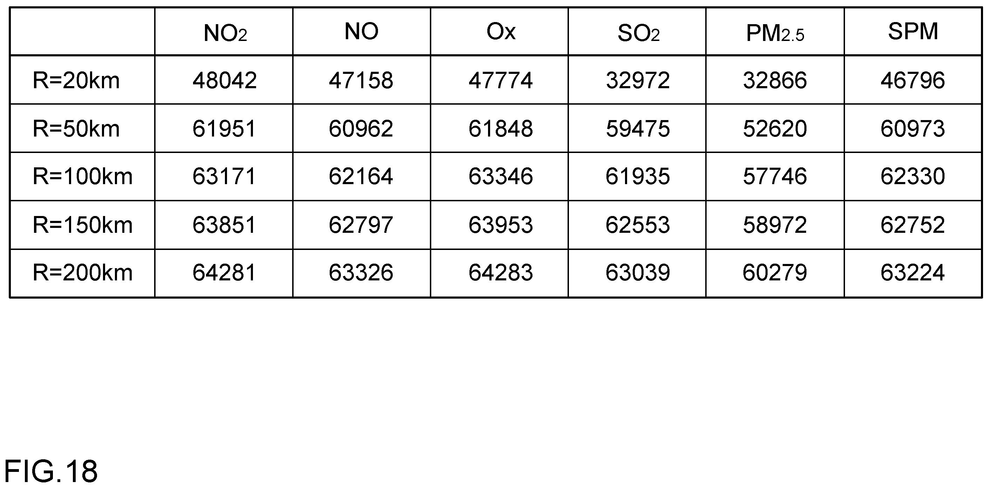

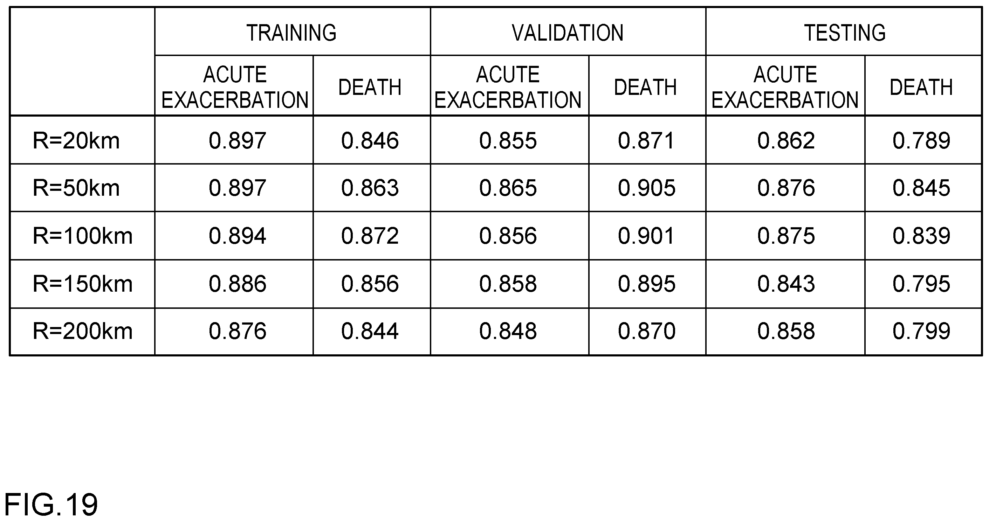

FIGS. 18 and 19 are explanatory diagrams showing the relationship between the upper limit value of a straight line distance from the current address of a patient to a measurement station (upper limit distance R) and the prediction accuracy. As mentioned above, in the present example, in a case where a measurement station is not present in the range of a predetermined upper limit distance R from the current address of the patient, there is a data absence for the environmental factors obtained by referencing the measurement data of a measurement station. FIG. 18 shows, for each environmental factor, the change in the number of data items (number of data items without data absences: 68,261 items (people·months)) associated as the upper limit distance R changes. As shown in FIG. 18, as the value of the upper limit distance R increases, the number of data absences for each environmental factor decreases.

FIG. 19 shows the change in the prediction accuracy of acute exacerbation and death that accompanies a change in the upper limit distance R. Irrespective of whether the training data, validation data, or test data is used, the prediction accuracy is highest when the upper limit distance R is 50 km or 100 km. If the upper limit distance R is too large, although data absences are reduced, the deviation from the environment to which the patient is actually exposed increases, which is thought to decrease the prediction accuracy. From the result shown in FIG. 19, it can be said that the upper limit distance R is preferably 20 km or more and 100 km or less.

A-6. Effects of the Present Embodiment:

As described above, the information processing device 100 of the present embodiment is a device for predicting the prognosis of a subject patient affected by a disease, and includes the model acquisition unit 113, the subject patient information acquisition unit 114, and the prognosis prediction execution unit 119. The model acquisition unit 113 acquires the prognosis prediction model MO, being a machine learning model that takes time-series information indicating a time-series transition of factors of the disease as an input, and outputs a prognosis of the disease. The subject patient information acquisition unit 114 acquires time-series information about the subject patient. The prognosis prediction execution unit 119 executes prognosis prediction of the subject patient using the time-series information about the subject patient and the prognosis prediction model MO, and outputs a result of the prognosis prediction.

In this way, according to the information processing device 100 according to the present embodiment, it is possible to predict the prognosis of a disease for each individual patient based on time-series information indicating time-series transitions of the factors of the disease. Therefore, according to the information processing device 100 of the present embodiment, the prognosis of the disease can be predicted with high accuracy.

In the present embodiment, the factors of the disease include an environmental parameter. According to the information processing device 100 of the present embodiment, by using information indicating a time-series transition of an environmental factor that could have a significant effect on the prognosis of the disease, the prognosis of the disease can be predicted with higher accuracy.

In the present embodiment, the factors of the disease include an environmental factor of a place of residence of the subject patient. According to the information processing device 100 of the present embodiment, for example, compared to a case where an environmental factor of the location of the hospital that the subject patient visits is used, by using information indicating a time-series transition of a factor of the environment to which the subject patient is primarily exposed, the prognosis of the disease can be predicted with even higher accuracy.

In the present embodiment, the environmental factor includes at least one of a presence status of an environmental pollutant, and a meteorological parameter. According to the information processing device 100 of the present embodiment, by using information indicating a time-series transition of an environmental factor that could have a significant effect on the prognosis of the disease, the prognosis of the disease can be predicted with even higher accuracy.

In the present embodiment, the time-series information includes information indicating an amount of change in the environmental factor. According to the information processing device 100 of the present embodiment, by using information indicating an amount of change in an environmental factor that could have a significant effect on the prognosis of the disease, the prognosis of the disease can be predicted with even higher accuracy.

In the present embodiment, the time-series information includes information that specifies values of the factors of the disease at a fixed time interval. According to the information processing device 100 of the present embodiment, compared to a case where information that irregularly specifies values of the factors of the disease is used, the prognosis of the disease can be predicted with even higher accuracy.

In the present embodiment, the time-series information includes information that specifies values of the factors of the disease at least every month. According to the information processing device 100 of the present embodiment, by using time-series information indicating monthly changes in the factors of the disease, the prognosis of the disease can be predicted with even higher accuracy.

In the present embodiment, the prognosis of the disease includes the occurrence of a plurality of events that are in a competing risk relationship, and the prognosis prediction model MO is a model trained using a machine learning algorithm corresponding to the plurality of events that are in a competing risk relationship. According to the information processing device 100 of the present embodiment, the occurrence of a plurality of events that are in a competing risk relationship can be predicted with high accuracy.

In the present embodiment, the prognosis prediction model MO outputs, as the prognosis of the disease, an index value representing the possibility of an event occurring among the plurality of events that are in a competing risk relationship. According to the information processing device 100 of the present embodiment, the occurrence of a plurality of events that are in a competing risk relationship can be predicted with high accuracy.

In the present embodiment, the plurality of events that are in a competing risk relationship include acute exacerbation and death. According to the information processing device 100 of the present embodiment, the occurrence of each of acute exacerbation and death, which are in a competing risk relationship, can be predicted with high accuracy.

In the present embodiment, the disease is a disease of the respiratory system or the circulatory system. According to the information processing device 100 of the present embodiment, by using time-series information indicating a time-series transition of the factors of a disease of the respiratory system or the circulatory system, it is possible to predict the prognosis of a disease of the respiratory system or the circulatory system with high accuracy.

In the present embodiment, the prognosis prediction execution unit 119 executes a hypothetical prognosis prediction of the subject patient using hypothetical information, in which a portion of the time-series information about the subject patient has been changed, and the prognosis prediction model MO, and predicts an effect of an intervention corresponding to the change based on an actual prognosis prediction result and a hypothetical prognosis prediction result, and outputs a prediction result of the effect of the intervention. According to such a configuration, it is possible to determine whether or not to actually perform an intervention based on an output prediction result of the effect of the intervention.

B. Modifications:

The technique disclosed herein is not limited to the embodiment described above, and can be modified in various forms without departing from the gist of this specification, and, for example, the following modifications can be made.

The configuration of the information processing device 100 according to the embodiment described above is merely an example, and various modifications are possible. The content of the prognosis prediction model acquisition processing and the prognosis prediction processing in the embodiment described above is merely an example, and various modifications are possible. For example, in the embodiment described above, although the information processing device 100 acquires the prognosis prediction model MO by creating the prognosis prediction model MO, the information processing device 100 may acquire a prognosis prediction model MO that has been generated by another device.

The factors (feature amounts) of the disease used to create the prognosis prediction model MO according to the embodiment described above, and the machine learning algorithm are merely examples, and various modifications are possible. For example, as the feature amounts used to create the prognosis prediction model MO, feature amounts other than the feature amounts illustrated in the embodiment described above may be used, or some of the feature amounts illustrated in the embodiment described above may be omitted. The machine learning algorithm used to create the prognosis prediction model MO may be an algorithm other than Dynamic-DeepHit or LSTM.

In the embodiment described above, although the information processing for predicting the prognosis of a patient affected by interstitial lung disease has been illustrated, the technique disclosed herein is not limited to interstitial lung disease, and can also be similarly applied as appropriate to predicting the prognosis of a patient affected by a disease. Because the prognosis of a disease of the respiratory system or the circulatory system is considered to be significantly affected by environmental factors, it is preferable that the disease factors used in prognosis prediction of a disease of the respiratory system or the circulatory system includes an environmental factor.

In the embodiment described above, a portion of the configuration realized by hardware may be replaced with software, or conversely, a portion of the configuration realized by software may be replaced with hardware.

DESCRIPTION OF REFERENCE NUMERALS

100 Information processing device

110 Control unit

111 Raw information acquisition unit

112 Learning data acquisition unit

113 Model acquisition unit

114 Subject patient information acquisition unit

119 Prognosis prediction execution unit

120 Storage unit

130 Display unit

140 Operation input unit

150 Interface unit

190 Bus

CP Prognosis prediction program

LD Learning data

MO Prognosis prediction model

RD Prognosis prediction result data

## Claims

1. An information processing device for predicting a prognosis of a subject patient affected by a disease, comprising:

   a model acquisition unit that acquires a prognosis prediction model, being a machine learning model that takes time-series information indicating a time-series transition of factors of the disease as an input, and outputs a prognosis of the disease;
   a subject patient information acquisition unit that acquires the time-series information about the subject patient; and
   a prognosis prediction execution unit that executes prognosis prediction of the subject patient using the time-series information about the subject patient and the prognosis prediction model, and outputs a result of the prognosis prediction.

2. The information processing device according to claim 1, wherein
   the factors of the disease include an environmental factor.

3. The information processing device according to claim 2, wherein
   the factors of the disease include an environmental factor of a place of residence of the subject patient.

4. The information processing device according to claim 3, wherein
   the environmental factor of the place of residence of the subject patient is an environmental factor of a point within a straight line distance of 200 km from a current address of the subject patient.

5. The information processing device according to claim 2 or 3, wherein
   the environmental factor includes at least one of a presence status of an environmental pollutant, and a meteorological parameter.

6. The information processing device according to claim 2 or 3, wherein
   the time-series information includes information indicating an amount of change in the environmental factor.

7. The information processing device according to claim 1, wherein
   the time-series information is information that specifies values of the factors of the disease at a fixed time interval.

8. The information processing device according to claim 7, wherein

the time-series information includes information that specifies values of the factors of the disease at least every month.

9. The information processing device according to claim 1, wherein

   the prognosis of the disease includes an occurrence of a plurality of events that are in a competing risk relationship, and
   the prognosis prediction model is a model trained using a machine learning algorithm corresponding to the plurality of events that are in a competing risk relationship.

10. The information processing device according to claim 9, wherein
    the prognosis prediction model is a model that outputs, as the prognosis of the disease, an index value representing a possibility of an event occurring among the plurality of events that are in a competing risk relationship.

11. The information processing device according to claim 9 or 10, wherein
    the plurality of events that are in a competing risk relationship include acute exacerbation and death.

12. The information processing device according to claim 1, wherein
    the disease is a disease of a respiratory system or a circulatory system.

13. The information processing device according to claim 1, wherein
    the prognosis prediction execution unit executes a hypothetical prognosis prediction of the subject patient using hypothetical information, in which a portion of the time-series information about the subject patient has been changed, and the prognosis prediction model, and predicts an effect of an intervention corresponding to the change based on an actual prognosis prediction result and a hypothetical prognosis prediction result, and outputs a prediction result of the effect of the intervention.

14. An information processing method for predicting a prognosis of a subject patient affected by a disease, comprising the steps of:

    acquiring a prognosis prediction model, being a machine learning model that takes time-series information indicating a time-series transition of factors of the disease as an input, and outputs a prognosis of the disease;
    acquiring the time-series information about the subject patient; and
    executing prognosis prediction of the subject patient using the time-series information about the subject patient and the prognosis prediction model, and outputting a result of the prognosis prediction.

15. A computer program for predicting a prognosis of a subject patient affected by a disease, which causes a computer to execute the processing of:

acquiring a prognosis prediction model, being a machine learning model that takes time-series information indicating a time-series transition of factors of the disease as an input, and outputs a prognosis of the disease;

acquiring the time-series information about the subject patient; and

executing prognosis prediction of the subject patient using the time-series information about the subject patient and the prognosis prediction model, and outputting a result of the prognosis prediction.

FIG.1

FIG.2

ACUTE EXACERBATION: XX% RISK REDUCTION
DEATH: YY% RISK REDUCTION

FIG.3

PROGNOSIS PREDICTION MODEL
ACQUISITION PROCESSING

ACQUIRE RAW INFORMATION Io — S110

PERFORM PREPROCESSING
(ESTIMATION, OUTLIER REMOVAL,
DATA AUGMENTATION) — S120

CREATE PROGNOSIS PREDICTION MODEL MO
USING MACHINE LEARNING — S130

END

FIG.4

FACTORS (FEATURE AMOUNTS) (44)

| PATIENT BACKGROUND (12) | AGE, BMI, GAP SCORE, CURRENT SMOKER, FORMER SMOKER, IPF, PPFE, SSc, COLLAGEN DISEASE, SEX, CCI≥3, mMRC≥2 |
| --- | --- |
| EXAMINATION FINDINGS (18) | H, BNP, WBC, NEUTROPHILS, LYMPHOCYTES, EOSINOPHILS, ALBUMIN, KL-6, SP-D, FVC(%pred), $FEV_1$(pred), $DL_{CO}$(%pred), 6-MINUTE WALKING DISTANCE, $PCO_2$, $PO_2$, $SpO_2$ MINIMUM VALUE, logCRP, CT IMAGE UIP PATTERN |
| ENVIRONMENTAL FACTORS (10) | $NO_2$, NO, $SO_2$, $PM_{2.5}$, SPM, PRECIPITATION, TEMPERATURE, SEASON (AUTUMN, SUMMER, WINTER) |
| TREATMENT INFORMATION (4) | PREDNISOLONE, CALCINEURIN INHIBITORS, IMMUNOSUPPRESSANTS, ANTIFIBROTIC AGENTS |

FIG.5

FIG.6

## FIG.7

EP 4 614 519 A1

LD

| TIME-SERIES INFORMATION | CORRECT LABEL | | |
|---|---|---|---|
| | SURVIVAL | ACUTE EXACERBATION | DEATH |
| FIRST MONTH DATA | 1 | 0 | 0 |
| SECOND MONTH DATA | 1 | 0 | 0 |
| THIRD MONTH DATA | 1 | 0 | 0 |
| ⋮ | ⋮ | | |
| (S-1)TH MONTH DATA | 1 | 0 | 0 |
| STH MONTH DATA | 0 | 1 | 0 |
| (S+1)TH MONTH DATA | 0 | 1 | 0 |
| ⋮ | ⋮ | | |
| TTH MONTH DATA | 0 | 1 | 0 |
| 0 padding | 0 padding | | |
| ⋮ | ⋮ | | |

FIG.8

EP 4 614 519 A1

```
        ┌─────────────────────────────────────┐
        │   PROGNOSIS PREDICTION PROCESSING    │
        └─────────────────────────────────────┘
                          │
                          ▼
      ┌──────────────────────────────────────────┐
      │  ACQUIRE SUBJECT PATIENT INFORMATION Ip   │──── S310
      └──────────────────────────────────────────┘
                          │
                          ▼
      ┌──────────────────────────────────────────┐
      │            PREDICT PROGNOSIS              │──── S320
      │  USING PROGNOSIS PREDICTION MODEL MO      │
      └──────────────────────────────────────────┘
                          │
                          ▼
      ┌──────────────────────────────────────────┐
      │         OUTPUT PREDICTION RESULT          │──── S330
      └──────────────────────────────────────────┘
                          │
                          ▼
              ┌───────────────────────┐
              │          END          │
              └───────────────────────┘
```

FIG.9

| Time＝12 | INTERNAL VALIDITY VERIFICATION | | EXTERNAL VALIDITY VERIFICATION | |
|---|---|---|---|---|
| | ACUTE EXACERBATION | DEATH | ACUTE EXACERBATION | DEATH |
| T=6 | 0.976 | 0.896 | 0.956 | 0.914 |
| T=12 | 0.945 | 0.896 | 0.957 | 0.910 |
| T=24 | 0.949 | 0.890 | 0.883 | 0.882 |
| T=36 | 0.949 | 0.875 | 0.850 | 0.872 |

Time      T

AT DIAGNOSIS    PREDICTION POINT      FUTURE PREDICTION

FIG.10

INTERNAL VALIDITY VERIFICATION

| Time＝12 | ACUTE EXACERBATION | | DEATH | |
|---|---|---|---|---|
| ENVIRONMENTAL FACTOR | MONTHLY CONCENTRATION | AMOUNT OF CHANGE | MONTHLY CONCENTRATION | AMOUNT OF CHANGE |
| T=6 | 0.977 | 0.959 | 0.896 | 0.903 |
| T=12 | 0.945 | 0.952 | 0.896 | 0.908 |
| T=24 | 0.949 | 0.929 | 0.890 | 0.908 |
| T=36 | 0.949 | 0.921 | 0.875 | 0.901 |

EXTERNAL VALIDITY VERIFICATION

| Time＝12 | ACUTE EXACERBATION | | DEATH | |
|---|---|---|---|---|
| ENVIRONMENTAL FACTOR | MONTHLY CONCENTRATION | AMOUNT OF CHANGE | MONTHLY CONCENTRATION | AMOUNT OF CHANGE |
| T=6 | 0.956 | 0.935 | 0.914 | 0.906 |
| T=12 | 0.957 | 0.894 | 0.910 | 0.899 |
| T=24 | 0.883 | 0.864 | 0.882 | 0.883 |
| T=36 | 0.850 | 0.824 | 0.872 | 0.872 |

FIG.11

INTERNAL VALIDITY VERIFICATION

| Time＝24 | ACUTE EXACERBATION | | DEATH | |
|---|---|---|---|---|
| ENVIRONMENTAL FACTOR | MONTHLY CONCENTRATION | AMOUNT OF CHANGE | MONTHLY CONCENTRATION | AMOUNT OF CHANGE |
| T=6 | 0.965 | 0.954 | 0.918 | 0.912 |
| T=12 | 0.970 | 0.938 | 0.905 | 0.905 |
| T=24 | 0.972 | 0.944 | 0.896 | 0.904 |
| T=36 | 0.967 | 0.917 | 0.885 | 0.896 |

EXTERNAL VALIDITY VERIFICATION

| Time＝24 | ACUTE EXACERBATION | | DEATH | |
|---|---|---|---|---|
| ENVIRONMENTAL FACTOR | MONTHLY CONCENTRATION | AMOUNT OF CHANGE | MONTHLY CONCENTRATION | AMOUNT OF CHANGE |
| T=6 | 0.903 | 0.940 | 0.933 | 0.899 |
| T=12 | 0.900 | 0.918 | 0.914 | 0.894 |
| T=24 | 0.865 | 0.878 | 0.904 | 0.900 |
| T=36 | 0.825 | 0.856 | 0.900 | 0.895 |

# FIG.12

|  | ACUTE EXACERBATION | DEATH |
|---|---|---|
| ACUTE EXACERBATION MODEL | 0.871 | - |
| DEATH MODEL | - | 0.889 |
| COMPETITIVE MODEL | 0.870 | 0.891 |

FIG.13

FIG.14

| | $W_{SURVIVAL}$ | $W_{ACUTE\ EXACERBATION}$ | $W_{DEATH}$ | Balanced-Accuracy | $F1_{SCORE}$ |
|---|---|---|---|---|---|
| k=0 | 1.00 | 1.00 | 1.00 | 0.631 | 0.614 |
| k=1 | 0.37 | 11.98 | 5.23 | 0.631 | 0.400 |
| k=2 | 0.13 | 143.40 | 27.37 | 0.434 | 0.181 |
| k=3 | 0.049 | 1717.27 | 143.21 | 0.342 | 0.076 |

FIG.15

FIG.16

FIG.17

| | NO$_2$ | NO | Ox | SO$_2$ | PM$_{2.5}$ | SPM |
|---|---|---|---|---|---|---|
| R=20km | 48042 | 47158 | 47774 | 32972 | 32866 | 46796 |
| R=50km | 61951 | 60962 | 61848 | 59475 | 52620 | 60973 |
| R=100km | 63171 | 62164 | 63346 | 61935 | 57746 | 62330 |
| R=150km | 63851 | 62797 | 63953 | 62553 | 58972 | 62752 |
| R=200km | 64281 | 63326 | 64283 | 63039 | 60279 | 63224 |

FIG.18

| | TRAINING | | VALIDATION | | TESTING | |
|---|---|---|---|---|---|---|
| | ACUTE EXACERBATION | DEATH | ACUTE EXACERBATION | DEATH | ACUTE EXACERBATION | DEATH |
| R=20km | 0.897 | 0.846 | 0.855 | 0.871 | 0.862 | 0.789 |
| R=50km | 0.897 | 0.863 | 0.865 | 0.905 | 0.876 | 0.845 |
| R=100km | 0.894 | 0.872 | 0.856 | 0.901 | 0.875 | 0.839 |
| R=150km | 0.886 | 0.856 | 0.858 | 0.895 | 0.843 | 0.795 |
| R=200km | 0.876 | 0.844 | 0.848 | 0.870 | 0.858 | 0.799 |

FIG.19

EP 4 614 519 A1

**EP 4 614 519 A1**

## INTERNATIONAL SEARCH REPORT

| | International application No. |
|---|---|
| | **PCT/JP2023/039023** |

**A. CLASSIFICATION OF SUBJECT MATTER**

*G16H 50/50*(2018.01)i
FI: G16H50/50

According to International Patent Classification (IPC) or to both national classification and IPC

**B. FIELDS SEARCHED**

Minimum documentation searched (classification system followed by classification symbols)

G16H50/50

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-144471 A (UNIV TOKAI) 10 September 2020 (2020-09-10) paragraphs [0020], [0022]-[0024], [0038], [0041], [0049]-[0054] | 1, 7-11, 14-15 |
| Y | | 2-6, 12-13 |
| Y | US 2016/0314256 A1 (RECIPROCAL LABS CORPORATION (D/B/A PROPELLER HEALTH)) 27 October 2016 (2016-10-27) paragraphs [0075]-[0081], claim 8 | 2-6, 12 |
| A | | 1, 7-11, 13-15 |
| Y | WO 2021/182595 A1 (KYOCERA CORPORATION) 16 September 2021 (2021-09-16) paragraphs [0083]-[0084], fig. 8 | 13 |
| A | | 1-12, 14-15 |
| A | WO 2021/205828 A1 (THE UNIVERSITY OF TOKYO) 14 October 2021 (2021-10-14) entire text, all drawings | 1-15 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **14 November 2023** | **28 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

34

## INTERNATIONAL SEARCH REPORT
### Information on patent family members

International application No.

**PCT/JP2023/039023**

| Patent document cited in search report | | | Publication date (day/month/year) | Patent family member(s) | | | Publication date (day/month/year) |
|---|---|---|---|---|---|---|---|
| JP | 2020-144471 | A | 10 September 2020 | (Family: none) | | | |
| US | 2016/0314256 | A1 | 27 October 2016 | US | 2020/0321127 | A1 | |
| | | | | paragraphs [0075]-[0081] | | | |
| | | | | US | 2022/0172845 | A1 | |
| | | | | WO | 2016/172614 | A1 | |
| WO | 2021/182595 | A1 | 16 September 2021 | US | 2023/0111852 | A1 | |
| | | | | paragraphs [0126]-[0127], fig. 8 | | | |
| | | | | EP | 4119056 | A1 | |
| | | | | CN | 115279270 | A | |
| | | | | AU | 2021234765 | A1 | |
| WO | 2021/205828 | A1 | 14 October 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **QI WU**. A Clinical Model for the Prediction of Acute Exacerbation Risk in Patients with Idiopathic Pulmonary Fibrosis. *BioMed Research International*, 2020, 1-6

- DeepHit: A Deep Learning Approach to Survival Analysis with Competing Risks. **LEE CHANGHEE**. Proceedings of the 31st AAAI Conference on Artificial Intelligence. Association for the Advancement of Artificial Intelligence, 2018, 2314-2321